# EUROPÄISCHE PATENTANMELDUNG

(11) **EP 0 765 640 A2**
(43) Veröffentlichungstag der Anmeldung: **02.04.1997**
(21) Anmeldenummer: 96115596.7
(22) Anmeldetag: 27.09.1996
(51) Int. Cl.: A61C 3/02

(54) **Bohrer zur Herstellung von Knochenkavitäten für zahnärztliche Implantate**

(30) Priorität: 30.09.1995 DE 19536716
(71) Anmelder: Lauks, Nikola, Dr., D-22397 Hamburg (DE)
(72) Erfinder: Lauks, Nikola, Dr., D-22397 Hamburg (DE)
(74) Vertreter: Gosch, Wolf-Dietrich

(57) **Zusammenfassung**

Bohrer zur Herstellung von Knochenkavitäten für zahnärztliche Implantate mit einem in ein Winkelstück einzurastenden Schaft (1) und einem diesem angewandten Knochenmaterial abtragenden Schneidkopf (2), wobei zwischen dem Schaft (1) und dem Schneidkopf (2) ein diesen tragender Bohrerkörper (3) angeordnet ist, dessen Querschnitt geringere Abmessungen aufweist als eine vom Schneidkopf (2) gebohrte Knochenkavität.

## Beschreibung

Die Erfindung betrifft einen Bohrer zur Herstellung von Knochenkavitäten für zahnärztliche Implantate mit einem in ein Winkelstück einzurastenden Schaft und einem diesem abgewandten, Knochenmaterial abtragenden Schneidkopf.

Derartige Bohrer werden üblicherweise verwendet, um eine Knochenkavität in einem menschlichen Kiefer zur Einbringung eines Implantates zu schaffen. Üblicherweise haben die Bohrer einen sich zwischen Schneidkopf und Schaft erstreckenden Bohrkörper, dessen Querschnitt mit demjenigen des Schneidkopfes identisch ist. Dies führt dazu, daß die bereits gebohrte Knochenkavität in feste Anlage an den Bohrkörper gelangt, da aufgrund der im Kieferknochen vorhandenen Druckverhältnisse der Knochen im Bereich der bereits gebohrten Kavität dazu neigt, sich in Richtung auf die Kavität auszudehnen und dabei den Bohrkörper des Bohrers zu beaufschlagen. Dies hat mehrere nachteilige Auswirkungen. Zum einen entsteht eine heftige Reibung zwischen dem Bohrkörper und dem ihn umgebenden Knochenmaterial. Durch diese Reibung wird Reibungshitze erzeugt, die äußerst nachteilig für das später erwünschte Einheilen des Implantates ist, da das Knochengewebe durch die Wärmentwicklung traumatisiert wird. Ferner entsteht durch die Anlage des Bohrerkörpers im Knochenmaterial ein unerwünschter Rütteleffekt, der die Präzision der in dem Kiefer einzubringenden Bohrung behindert. Auch wird der Abfluß des abgetragenen Knochenmaterials bzw. der Knochenspäne behindert, da nicht ausreichend Raum zwischen dem Bohrerkörper und dem Kieferknochen zur Verfügung steht, daß das abgetragene Material bzw. auch zusätzlich eingebrachte Spülflüssigkeit unbehindert abfließen können.

Aufgabe der vorliegenden Erfindung ist es daher, einen Bohrer der eingangs genannten Art zu schaffen, mit dessen Hilfe präzise einem einzubringenden Implantat vollständig angepaßte Knochenkavitäten ohne Traumatisierung des Knochengewebes geschaffen werden können.

Diese Aufgabe wird erfindungsgemäß dadurch gelöst, daß zwischen dem Schaft und dem Schneidkopf ein diesen tragender Bohrerkörper angeordnet ist, dessen Querschnitt geringere Abmessungen aufweist, als eine vom Schneidkopf gebohrte Knochenkavität.

Durch den zwischen dem Bohrerkörper und dem verbleibenden Knochenmaterial entstehenden Ringspalt wird zunächst jede Reibung zwischen Bohrerkörper und Knochenmaterial verhindert, weil der Bohrerkörper nicht mit dem Knochenmaterial in Berührung kommt. Ein Rütteleffekt kann nicht entstehen. Auch wird das Knochenmaterial durch Reibungshitze nicht erwärmt und der Bohrer hat einen wesentlich geringeren Reibungswiderstand zu überwinden als ein herkömmlicher Bohrer. Durch den zwischen Bohrerkörper und dem ihm umgebenden Knochenmaterial entstehenden Ringspalt kam sowohl das abgetragene Knochenmaterial wie auch die zur Spülung und Kühlung durch den Bohrer herangeführte Ringerlösung zuverlässig abgeleitet werden.

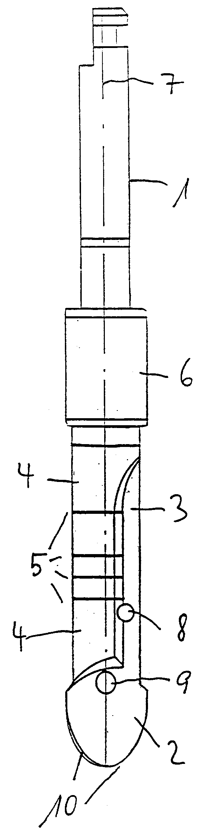

Weitere Einzelheiten der Erfindung ergeben sich aus der nachfolgenden Ausführlichen Beschreibung und der beigefügten Zeichnung, in denen eine bevorzugte Ausführungsform der Erfindung beispielsweise veranschaulicht ist.

Die Zeichnung zeigt einen erfindungsgemäßen Bohrer in schematischer Darstellung in Seitenansicht.

Ein erfindungsgemäßer Bohrer besteht im wesentlichen aus einem in ein Winkelstück einzurastenden Schaft 1 und an seinem diesem abgewandten Ende vorgesehenen Knochenmaterial abtragenden Schneidkopf 2, zwischen denen sich ein den Schneidkopf 2 tragender Bohrerkörper 3 erstreckt, dessen Querschnitt geringere Abmessungen aufweist als eine vom Schneidkopf 2 gebohrte Knochenkavität. Der Schneidkopf 2 überragt den Bohrerkörper 3 in seinen Querrichtungen. Der Bohrerkörper 3 kann einen eckigen, zum Beispiel quadratischen Querschnitt aufweisen, dabei sind dem Schneidkopf 2 benachbarte Bereiche 4 mit ihren Querschnitt reduzierenden Abfasungen versehen. Der Bohrerkörper 3 kann auch einen runden Querschnitt aufweisen.

Der Bohrerkörper 3 ist mit Markierungsringen 5 versehen, die eine Eindringtiefe des Bohrers in das Knochenmaterial anzeigen.

Zwischen dem Bohrerkörper 3 und dem Schaft 1 kann ein Flansch 6 angeordnet sein, dessen Abmessungen im Sinne einer drehbeweglichen Lagerung in einer Bohrbuchsenschablone angepaßt ist, wie sie beispielsweise in der europäischen Patentschrift 0328911 beschrieben ist.

Der Schaft 1, der Flansch 6 und der Bohrerkörper 3 sind in ihren Längsrichtungen von einer Spülmittelleitung 7 durchzogen, deren Mündungen 8, 9 seitlich im Bohrerkörper 3 und benachbart des Schneidkopfes 2 angeordnet sind. Dabei ist die im Bereich des Schneidkopfes 2 vorgesehene Mündung 9 in seinem seinen Schneitkanten 10 abgewandten Bereichen angeordnet. Der Schneidkopf 2 ist vorzugsweise blattförmig ausgebildet, um eine möglichst geringe auf das zu bohrende Knochenmaterial aufliegende Auflagefläche aufzuweisen.

Die Form und die Abmessungen des Schneidkopfes 2 entsprechen vorzugsweise denjenigen eines Endbereiches eines in die zu bohrende Kavität einzusetzenden Implantats.

Einem Bohrer ist vorzugsweise eine Serie weiterer Bohrer zugeordnet, bei denen die Abmessungen der Schneidköpfe 2 im Sinne einer abgestuften Erweiterung der Bohrkavitäten einander angepaßt sind. Dadurch wird die Wirkungsweise des Bohrers noch weiter verbessert. Durch Auswechseln der einzelnen Bohrer nach einer Pilotbohrung jeweils um eine Stufe stärker wird die Knochenkavität erweitert, so daß es auch nur zu geringfügiger Traumatisierung des Knochengewebes kommt, weil eine Verklemmung des Bohrers in der Kavität zuverlässig verhindert wird, der Kraftaufwand des Behandlers gering bleibt und die Gefahr der Herstellung einer unpräzisen und traumatischen Kavität beseitigt wird.

## Patentansprüche

1. Bohrer zur Herstellung von Knochenkavitäten für zahnärztliche Implantate mit einem in ein Winkelstück einzurastenden Schaft und einem diesem abgewandten, Knochenmaterial abtragenden Schneidkopf, dadurch gekennzeichnet, daß zwischen dem Schaft (1) und dem Schneidkopf (2) ein diesen tragender Bohrerkörper (3) angeordnet ist, dessen Querschnitt geringere Abmessungen aufweist als eine vom Schneidkopf (2) gebohrte Knochenkavität.

2. Bohrer nach Anspruch 1, dadurch gekennzeichnet, daß der Schneidkopf (2) den Bohrerkörper (3) in dessen Querrichtungen überragt.

3. Bohrer nach Anspruch 1 und 2, dadurch gekennzeichnet, daß der Bohrerkörper (3) einen eckigen Querschnitt aufweist.

4. Bohrer nach Anspruch 3, dadurch gekennzeichnet, daß in seinen dem Schneidkopf (2) benachbarten Bereichen (4) der Bohrerkörper (3) seinen Querschnitt reduzierende Abfasungen aufweist.

5. Bohrer nach Anspruch 1 und 2, dadurch gekennzeichnet, daß der Bohrerkörper (3) einen runden Querschnitt aufweist.

6. Bohrer nach Anspruch 1 bis 5, dadurch gekennzeichnet, daß der Bohrerkörper (3) mit Eindringtiefen des Bohrers anzeigenden Markierungsringen (5) versehen ist.

7. Bohrer nach Anspruch 1 bis 6, dadurch gekennzeichnet, daß zwischen dem Bohrerkörper (3) und dem Schaft (1) ein Flansch (6) angeordnet ist, dessen Abmessungen im Sinne einer drehbeweglichen Lagerung in einer Bohrbuchsenschablone dieser angepaßt ist.

8. Bohrer nach Anspruch 1 bis 7, dadurch gekennzeichnet, daß der Schaft (1), der Flansch (6) und der Bohrerkörper (3) von einer Spülmittelleitung (7) durchzogen sind, deren Mündungen (8), (9) im Bohrerkörper (3) und am Schneidkopf (2) angeordnet sind.

9. Bohrer nach Anspruch 1 bis 8, dadurch gekennzeichnet, daß die am Schneidkopf (2) vorgesehene Mündung (9) in seinen Schneidkanten (10) abgewandten Bereichen angeordnet ist.

10. Bohrer nach Anspruch 1 bis 9, dadurch gekennzeichnet, daß der Schneidkopf (2) blattförmig ausgebildet ist.

11. Bohrer nach Anspruch 1 bis 10, dadurch gekennzeichnet, daß der Schneidkopf (2) in seiner Form und seinen Abmessungen einem Endbereich eines in die zu erzeugende Kavität einzusetzenden Implantat entspricht.

12. Bohrer nach Anspruch 1 bis 10, dadurch gekennzeichnet, daß ihm eine Serie weiterer Bohrer zugeordnet ist, deren Abmessungen der Schneidköpfe (2) im Sinne einer abgestuften Erweiterung der Knochenkavitäten angepaßt sind.
